# EUROPEAN PATENT APPLICATION

(11) **EP 2 163 627 A1**
(43) Date of publication of application: **17.03.2010**
(21) Application number: 08016080.7
(22) Date of filing: 12.09.2008
(51) Int. Cl.: C12N 15/30, C07K 14/445, A61K 35/68

(54) **Attenuated liver-stage Plasmodium and vaccin containing the same**

(71) Applicant: Bernhard-Nocht-Institut für Tropenmedizin, 20359 Hamburg (DE)
(72) Inventor: Heussler, Volker, Dr., 22880 Wedel (DE); Horstmann, Sebastian, 22143 Hamburg (DE); Helm, Susanne, 22049 Hamburg (DE); Rennenberg, Annika, 20099 Hamburg (DE); Nagel, Andreas, 21360 Vögelsen (DE)
(74) Representative: Kröncke, Rolf

(57) **Abstract**

The present invention relates to a genetically engineered live Plasmodium organism. In particular, the present invention relates to an attenuated live Plasmodium organism characterized in being attenuated in the liver-stage. In a further aspect, the present invention relates to isolated nucleic acids relating to a liver specific promotor allowing liver-stage specific expression of mRNA in Plasmodium organisms. In addition, pharmaceutical compositions, in particular, vaccines, are provided comprising the live plasmodium organism of the present invention in particular useful for vaccination against malaria diseases.

## Description

The present invention relates to a genetically engineered live Plasmodium organism. In particular, the present invention relates to an attenuated live Plasmodium organism characterized in being attenuated in the liver-stage. In a further aspect, the present invention relates to isolated nucleic acids relating to a liver specific promotor allowing liver-stage specific expression of mRNA in Plasmodium organisms. In addition, pharmaceutical compositions, in particular, vaccines, are provided comprising the live Plasmodium organism of the present invention in particular useful for vaccination against Plasmodium liver infections, like malaria diseases.

### Background of the invention

Malaria as a typical example of parasitic protozoa induced diseases has a tremendous impact on human health, killing millions annually, and the disease is a major impediment for social and economic development of nations in malaria-endemic areas, in particular, in sub-Saharan Africa. Infection of protozoa of the genus plasmodium typically develops to the disease identified as malaria. Various forms of malaria exist, for example, malaria tropica is induced by infection with Plasmodium falciparum while malaria tertiana is induced by Plasmodium vivax. Plasmodium falciparum and Plasmodium vivax are the most common types of Plasmodium. Until recently, it was assumed that only four types of the Plasmodium parasite can infect humans, namely Plasmodium falciparum, Plasmodium vivax and also Plasmodium ovale and Plasmodium malariae. However, it has been described recently that Plasmodium knowlesi is also able to infect humans and Plasmodium knowlesi infections were identified as Plasmodium malariae infections before. Typically, this group of human pathogenic Plasmodium species is referred to as malaria parasites.

Malaria infection begins when the Anopheline female injects infective sporozoites into the mammalian host. The parasites multiply within red blood cells causing symptoms that include symptoms of anemia (light headedness, shortness of breath, tachycardia etc.) as well as other general symptoms such as fever, chills, nausea, flu-like illness, and in severe cases, coma and death.

In addition, other Plasmodium species are able to infect primates, rodents, birds and reptiles. For example, Plasmodium yoelli and Plasmodium berghei are parasites infecting rodents inducing a malaria like diseases pattern in mice and, thus, represents a good model for studying malaria.

Today about 500 million people are infected with Plasmodium parasites inducing malaria. About 1 million people, most of them children, die annually. The vast majority of malaria occur in children under the age of five years.

Treatment of malaria as a vector-borne infectious diseases caused by protozoan parasites is treated by the use of Plasmodium specific drugs, thus, preventing expansion of the parasites and further infection of cells in the host. There are several families of drugs used to treat malaria, for example chloroquine represents a very cheap and, until recently, very effective drug which represents the anti-malarial drug of choice for many years in most parts of the world. However, resistance of Plasmodium falciparum to chloroquine has spread recently from Asia to Africa and, moreover, chloroquine resistance is associated with reduced sensitivity to other anti-malarial drugs, such as quinine and amodiaquine. Chloroquine resistance or pyrimethamine resistance require the use of other drugs, for example, artemisinin derivatives or mefloquine. However, these drugs are very expensive and, thus, it is difficult to enable treatment in Africa and Asia due to the high costs. Further, various drugs are provided as prophylactic drugs in regions were malaria is endemic. However, these prophylactic regimen will also result in further development of resistance.

Prevention and disease control was also effected by eradicating the mosquitos as vectors of the parasites.

The life cycle of Plasmodium is very complex. Sporozoites from the saliva of a biting female mosquito are transmitted to either the blood or the lymphatic system of the recipient. Typically, the sporozoites migrate from the bloodstream to the liver and invate hepatocytes. In the liver, the sporozoites penetrate the endothel and invade hepatocytes and settling therein. Those sporozoites move into the parasitophorous vacuole created by invagination of the hepatocyte plasma membrane. Inside this compartment the sporozoite transforms into the liver-stage. The liver-stage grows rapidly and undergoes multiple rounds of nuclear division. The infected liver-cell or hepatocyte then leave the cell structure and so called merosome, representing vesicle filed with so called merozoites are liberated into the bloodstream. The mesosomes can reach the lung where they accumulate and release merozoites. The merozoites can infect red blood cells and after several rounds of amplification they break out of the infective blood cells and can invade further blood cells, thus, resulting in anaemia.

The parasites of the liver-stage are not able to infect further hepatocytes but sporozoites are required for infection of hepatocytes. Liver-stages are predicted to express many different proteins, some unique to this stage, but only few of those unique molecules have been identified insofar. For example in WO 2007/041216 Plasmodium liver-stage antigens are described which should allegedly be liver-stage specific. The identification of liver-stage specific molecules is important because the infected hepatocyte has been established as the primary target of the sterile protective immune response in the radiation attenuated sporozoites vaccine model and recently the genetically attenuated sporozoites vaccine model, an overview is given for example in Matuschweski (2006), Curr. Op. Immunol. 18:1-9. Typically, the liver-stage represents the preferred stage for protective immune response since hepatocytes are able to interact with the cells of the immune system.

Various strategies have been developed for providing prophylaxis or treatment of malaria using the liver stage as the target for vaccine and prophylactic drugs, since the parasite numbers are limited and successful intervention at this stage prevents infection. More than 40 years ago radiation attenuated parasites have been used for immunizing mice against malaria, e.g. Nusszweig et al. 1967, Nature, 216, 160-162. Recently new approaches have been developed for providing live attenuated Plasmodium vaccines based on knock out of specific genes which are expressed in the sporozoite stage and in the early liver-stage. For example, in WO 2006/062928 life genetically engineered protozoan vaccines are described wherein a stage specific gene function that is required by the protozoan parasite for establishing a secondary infection in the red blood host has been disrupted.

WO 2005/063991 describes life-genetically attenuated malaria vaccine for inoculating a vertebrate host for immunizing against malaria wherein a liver stage specific gene function has been disrupted.

Thus, radiation attenuated or genetically attenuated parasites arrests in liver stage development are considered as potent elicitors of immune responses that completely protect against infection. However, it is assumed that the attenuated parasites would result in an insufficient antigen presentation in the host cells, thus, not fully activating the immune system and, as a consequence, not enabling prophylactic vaccination. Further, when sporozoites are able to pass through the liver stage, development of merozoites is possible.

Thus, there is an ongoing need in the art for vaccines that protect against malaria infection and diseases. There is also a need in the art for genetically engineered attenuated live Plasmodium organism useful as vaccines against malaria infection. The present invention addresses these needs and others resulting in prevention of the erythrocytic stages and the development of malaria.

### Summary of the invention

In a first aspect, an isolated nucleic acid molecule or functional fragments or derivatives thereof is provided which is a liver stage specific promotor, thus, allowing liver stage specific expression of mRNA in parasitic protozoa. That is, the present inventors identified a new promotor allowing liver stage specific expression of mRNA. In another aspect, genetically engineered live attenuated Plasmodium organism are provided containing the nucleic acid molecule according to the present invention. Particularly preferred, said Plasmodium organisms are selected from Plasmodium falciparum, Plasmodium berghei, Plasmodium yoelli and Plasmodium vivax.

In addition, a pharmaceutical composition, preferably a vaccine, is provided containing said live Plasmodium organism according to the present invention or the isolated nucleic acid molecule according to the present invention.

A further aspect relates to a method for producing said genetically engineered live Plasmodium organism suitable for vaccination comprising the step of introducing nucleic acid molecules according to the present invention into the Plasmodium organism.

In another aspect, an isolated nucleic acid molecule or functional fragment or derivative thereof are provided as well as isolated polypeptides encoded by said nucleic acids relating to a polypeptide specifically expressed during the liver stage.

### Brief description of the figures

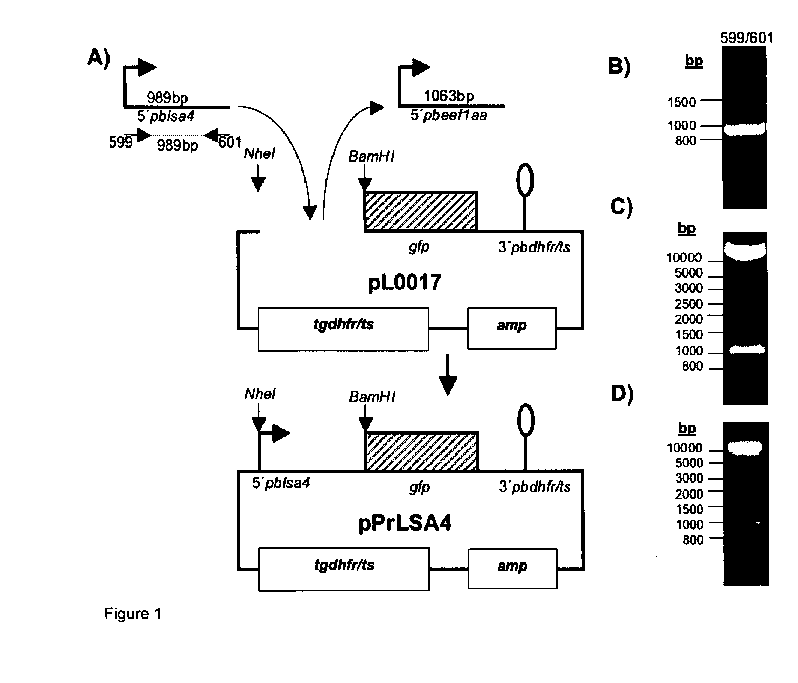

Figure 1: A) Schematic illustration of promotor subloning the 987bp promotor region of *pb103464* (5'pblsa4) was cloned in the Nhel/BamHl digested pI0017 vector in front of GFP to analyse promotor activity by immunofluorescence imaging in transfected *P. berghei.* B) PCR amplification of the 987 bp promotor fragment using primer 599 and 601. C) removal of the 1063 bp fragment pbeef1aa from the plasmid pL0017 C) control digest of the new vector pPrLSA4 (pL0017-pb103464-promotor) using NheI/BamHI.

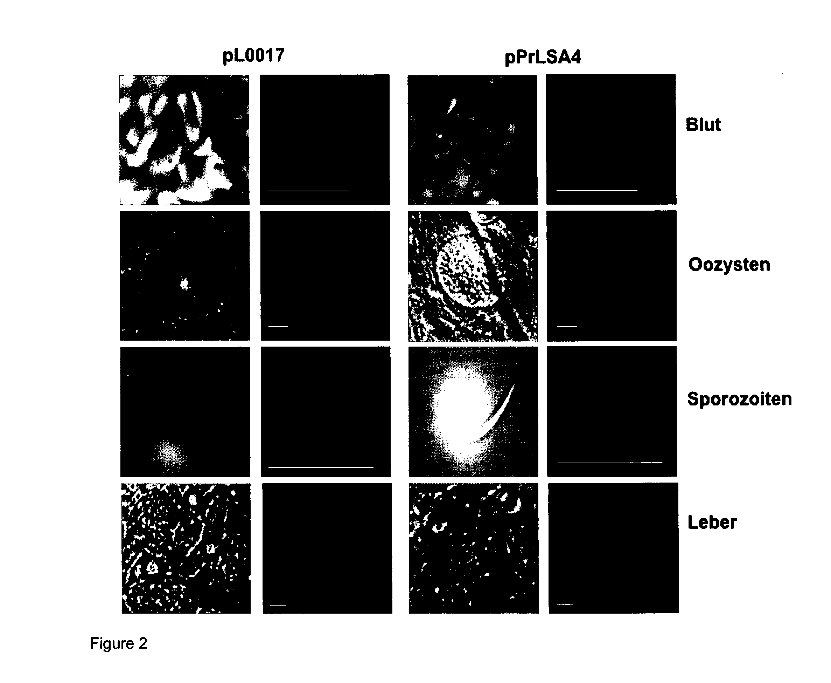

Figure 2: Comparison of GFP expression of transgene parasites transfected with pL0017 and pPrLSA4 (pL0017-pb103464-promotor). pL0017 contains a constitutive promotor and accordingly transfected parasites express GFP in all life cycle stage. pL0017-pb103464-promotor (pPrLSA4) contains the liver stage specific promotor and transfected parasites express GFP only in liver stages but not in blood and insect stages (oocysts, sporozoites).

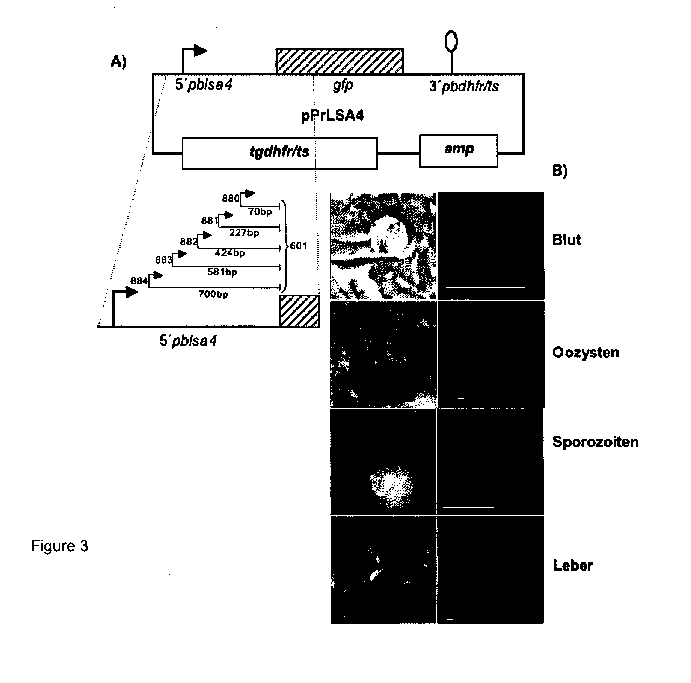

Figure 3: The 987 bp promotor fragment is nessesary to gurantee liver specific gene expression. Only the 987 promotor but not all other shorter promotor versions resulted in liver stage specific GFP expression. Shorter fragments produce GFP also in other stages (insect stages) indicating that negative regulator elements excist in the 987 bp promotor fragment.

### Detailed description of the present invention

In one aspect the present invention provides an isolated liver stage specific promotor nucleic acid of Seq. ID No. 1 or functional fragments or derivatives of said nucleic acid molecules of Seq. ID No. 1 allowing liver-stage specific expression of mRNA in Plasmodium organism.

That is, the present inventors identified a new promotor of Plasmodium, said promotor is a liver-stage specific promotor, thus, enabling liver-stage specific transcription and expression of nucleic acids and polypeptides, respectively.

In this connection, "attenuated" refer to the fact that said genetically engineered live Plasmodium organisms are inhibited from developing into the erythrocytic stage.

The term "heterologous peptide" as used herein refers to a heterologous peptide, like a toxin, which is not naturally expressed in the liver stage of Plasmodium infection. For example, the heterologous peptide or molecule is a toxin derived from prokaryotic or eukaryotic species. In addition, the heterologous peptide may be derived from a different Plasmodium species or the same Plasmodium species whereby said peptide is not a liver stage specific peptide.

The term "liver stage" refers to a specific development stage of the Plasmodium organism, restricted to the development of the parasite in a liver cell of the host. The liver stage begins with the invasion of a sporozoite into a liver-cell and ends with the release of merozoites from the merosomes. That is, e.g. in Biologie von Parasiten, Springer-Lehrbuch ,Lucius, Richard, Loos-Frank, Brigitte, Spektrum-Verlag, 2. Aufl., 2008, X, 552 S. 270 Abb.ISBN: 978-3-540-37707-8, the various life cycle stages of Plasmodium are illustrated.

The term "polypeptide" or "peptide" refers to a polymer of amino acids and does not refer to a specific length of the product; thus, peptides, oligopeptides, and proteins are included within the definition of polypeptide. Said polypeptide may also be post-expression modified, that is, may comprise glycolysations, acetylations, phosphorylations and the like. In addition, the polypeptide may contain one or more analogous of amino acid like unnatural amino acids, PNA, etc., polypeptides with substituted linkages, as well as other modifications known in the art, both naturally occurring and non-naturally occurring.

The term "immunogenic" refers to ability of a polypeptide to elicit a humoral and/or cellular immune response.

The term "isolated nucleic acid" or "isolated nucleic acid molecule" refers to a nucleic acid molecule, DNA or RNA, that has been removed from its native environment. For example, recombinant nucleic acids contain in a vector are considered isolated for the purposes of the present invention. Examples of isolated nucleic acid molecules include recombinant DNA molecules maintained in heterologous host cells or purified DNA molecules in solution. Isolated RNA molecules include in-vivo or in-vitro RNA transcripts of the DNA molecules of the present invention alternatively, the isolated nucleic acid molecules may be obtained by synthetic methods.

The term "functional fragment or functional derivative thereof" relates to fragments or derivatives of the isolated nucleic acid molecule of Seq. ID No. 1, insofar that the same allow liver stage specific transcription and expression of mRNA in a Plasmodium organism. Following, the approach provided in the example, the skilled person can verify whether the fragment or derivative represents a functional fragment or functional derivative of nucleic acids of Seq. ID No. 1.

The term "stage specific gene function" refers to the gene function that is restricted to one effective stage of the parasite for it to develop into a subsequent stage. For example, in Plasmodium, the term "liver stage specific gene function" refers to an expression that is restricted to liver stage parasites. But that is not required for entering into host hepatocytes or, preferably, maintenance of the parasite in asexual blood cell stages and production of infective sporozoites in mosquitos.

The phrase "disrupt a stage specific gene function" refers to interfering with a stage specific gene function such as to completely or partially inhibit, inactivate, attenuate, or block stage specific gene function, for example, by gene disruption or influencing transcription, translation, protein folding, and/or protein activity.

As used herein, the term "homologue" refers to a gene or a protein encoded by a gene, in one protozoan organism that has significant sequence identity over a large portion of the sequence to a gene, or protein encoded by a gene, in another protozoan organism. A homologue may be an orthologue. The term "orthologue" refers to the gene, or the protein encoded by a gene, in one protozoan organism that has the highest degree of sequence identity within the genome or proteome of the protozoan organism to a gene, or a protein encoded by a gene, in another protozoan organism. Thus, orthologous sequences may be homologoues sequences in different species that arose from a common ancestral sequence during specification.

Homologues of genes may be identified using standard methods including in-vitro methods such as using hybridization or PCR and in silico sequence comparison methods.

The present inventors identified new nucleic acid molecules representing a liver stage specific promotor in Plasmodium organism. In addition, a liver stage specific protein, herein referred to as Pb103464 encoded by the P. berghei gene pb103464.00.0 which is a homolog of the P.yoelii gene py05129 has been identified as a liver stage specific protein, being expressed exclusively in the liver stage, and claimed herein.

It has been recognized that the Pb103464 protein is expressed during late liver phase only and that expression of said late liver stage specific protein is controlled by the promotor according to the present invention.

In view of the stage specificity of the promotor, it is suggested to use said promotor for stage specific expression of polypeptides allowing attenuation of the Plasmodium organism. Said attenuation of the parasites may be attained by using proteins allowing the formation of pores in the parasitophorous vacuole (PV) of the parasite, thus, disintegrating the membrane of the parasitophorous vacuole and, as a consequence, attenuating the parasite by inhibiting further development of the parasite into the merozoite stage. For example, said peptide is a protein derived from Plasmodium containing the membrane-attack complex/perforin domain used by sporozoite to travel through cells as described by Kaiser et al., 2004, Mol Biochem Parasitol, 133, 15-26. An alternative of the nucleic acid molecule under the control of the liver stage specific promotor according to the present invention, encoding a heterologous peptide is an inhibitor of proteases, e.g. the protein inhibitor PbICP (P. berghei Inhibitor of cysteine protease). Inhibition of the protease, here the cysteine protease in the parasitophorous vacuole would inhibit the release of the merozoites present in the host cells by inhibition of the formation of the merosome.

Another possibility to attenuate the parasite in the liver stage is to disintegrate or perforate the parasitophorous vacuole membrane (PVM) using heterologous peptides.

Said heterologous peptides encoded nucleic acids under the control of liver stage specific promotor according to the present invention enables liver stage specific expression of said heterologous peptides.

Preferably, said heterologous peptides are toxins. Said toxins, for example bacteria derived lysis inducing peptides perforate the PVM, thus, inhibiting the development of merozoites.

For example, the protein listeriolysin O derived from Listeria monocytogenes represents a heterologous peptide useable according to the present invention. Listeriolysin O belongs to the family of the cholesterol dependent cytolysines, CDC, which forms a family of pore forming proteins. Mutants of said listeriolysin O which is normally active only in acid pH milieu also allow perforation of the cell membrane at neutral pH values. Said mutants are known in the art.

Further, toxins may be selected from Clostridium perfringens, Listeria monocytogenes, Yersinia pestis, Shigella dysenteriae, Escherichia coli, Plasmodium spec. Another alternative may be Perfringolysin O derived from Clostridium perfringens, Shiga toxin derived from Shigella dysenteriae, Escherichia coli enterotoxins, enterotoxin A derived from Clostridium botulinum type C, Yersinia outercoat proteins derived from Yersinia pestis.

Alternatively, the nucleic acid molecule under the control of the late liver stage promotor according to the present invention may be derived from non-procaryotic organism, e.g. may represent viral proteins or lysins described in prokaryotic or eukaryotic systems.

In a further aspect, the present invention provides a genetically engineered live Plasmodium organism containing the nucleic acid molecules according to the present invention. Said genetically engineered live Plasmodium organisms are attenuated live Plasmodium organism.

The genetically engineered attenuated live Plasmodium organism is preferably selected from the group of Plasmodium falciparum, Plasmodium vivax, Plasmodium malariae, Plasmodium ovale, Plasmodium knowlesi, Plasmodium berghei or Plasmodium yoelli.

These Plasmodium organisms according to the present invention or the isolated nucleic acid molecules according to the present invention are particular useful in pharmaceutical compositions. Hence, another aspect of the present invention relates to pharmaceutical compositions comprising the live attenuated genetically engineered Plasmodium organism according to the present invention and/or isolated nucleic acid molecule(s) according to the present invention, and, optionally, a diluent, carrier or excipient.

In a preferred embodiment of said pharmaceutical composition, the pharmaceutical composition is a vaccine suitable for prophylactic vaccination against malaria.

Hence, the present invention further provides a vaccine composition comprising a live attenuated genetically engineered protozoan parasite according to the present invention whereby the attenuated Plasmodium organism does not enter the erythrozytic stage of the protozoan parasite.

The dosage is empirically selected to achieve the desired immune response in the host. By "immune response" is meant an acquired and enhanced degree of protective immunity, preferably complete or sterile protection against subsequent exposure to wild type protozoan parasites.

The pharmaceutical composition of the present invention may further comprise a pharmaceutical acceptable carrier, excipient and/or diluent. Examples of suitable pharmaceutical carriers are well known in the art and include phosphate buffered saline solutions, sterile solutions etc. Compositions comprising such carriers can be formulated by well known conventional methods. These pharmaceutical compositions can be administered to the subject at a suitable dose. Administration of the suitable compositions may be effected by different ways, e. g., by intravenous, subcutaneous, intramuscular, topical, or intradermal administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The attenuated live Plasmodium may be administered in an amount of from 100 to 10.0000, like 300 to 5000, like 500 to 2000 sporozoites per dose; however, doses below or above this exemplary range are envisaged, especially considering the aforementioned factors.

Administration of the suitable compositions may be effected by different ways, e. g., by intravenous, intraperitoneal, subcutaneous, intramuscular, topical or intradermal administration.

Progress can be monitored by periodic assessment. The compositions of the invention may be administered locally or systemically. Administration will generally be parenterally, e. g., subcutaneously. Preparations for parenteral administration include sterile aqueous solutions, or suspensions. Aqueous carriers include suspensions, including saline and buffered media. Parenteral vehicles include sodium chloride solution, Ringer's dextrose, dextrose and sodium chloride, or lactated Ringer's. Intravenous vehicles include fluid and nutrient replenishers, electrolyte replenishers (such as those based on Ringer's dextrose), and the like.

Preservatives and other additives may also be present such as, for example, antimicrobials, anti-oxidants, cheating agents, and inert gases and the like.

Particularly preferred the pharmaceutical composition comprising the live attenuated genetically engineered Plasmodium organism is a living vaccine suitable for administration via the parenteral route, in particular, allowing subcutaneous injection of live attenuated parasites according to the present invention.

A further aspect relates to the production of said genetically engineered live Plasmodium organism according to the present invention suitable for vaccination comprising the step of introducing the isolated nucleic acid molecule according to the present invention into a live Plasmodium organism and, subsequently, selecting genetically engineered live Plasmodium organism containing the nucleic acid molecules.

Further, the present invention also provides methods for inducing an immune response against plasmodium parasites, in particular, Plasmodium parasites inducing malaria comprising administering a genetically engineered live attenuated Plasmodium organism according to the present invention. Methods for administering said Plasmodium organisms and inducing an immune response against Plasmodium parasites are described in the art. The skilled person is well aware about the specific requirements. Said method refers to therapeutic as well as prophylactic or preventive measures or treatments, wherein the object is to prevent or slow down the targeted pathologic condition or disease. Preferably, the method allows the prophylactic or preventive vaccination of individuals against malaria.

The method comprises the administration of an effective amount for the therapeutic or prophylactic treatment. In this connection, the term "effective amount" refers to amount or doses of a composition sufficient to induce the desired response, in particular, to induce an immunogenic response allowing vaccination of an individual to which it is administered. The effective amount and method of administration of a particular therapeutic or prophylactic treatment may vary based on the individual patients and the stage of the disease as well as other factors known to those of skilled in the art.

The exact dosage is chosen by the individual physician in view of the individual to be treated. Dosage and administration are adjusted to provide sufficient levels of the active moiety or to maintain the desired effect. Additional factors that may be taken into account include age and weight of the individual, diet, time and frequency, the way of administration, drug combination(s), reaction sensitivity and tolerance/response to therapy.

The pharmaceutical composition, in particular, the vaccine according to the present invention are particularly useful and superior over known live attenuated Plasmodium organism based vaccines since according to known vaccine regimens immunization with about 10.000 to 50.000 sporozoites is required, see e.g. Labaied et al., 2007, Mol Biochem Parasitol, 153, 158-166. Using the approach of knock out genetically modified Plasmodium organism wherein genes, in particular, liver stage expressed gene products, are knocked out, sporozoites may enter the hepatocytes. However, the amount of proteins expressed therein is low and, thus, large amounts of sporozoites are necessary. Furthermore, there is still the danger of "break through infection" that means that the gene is not completely knocked out but sporozoites are present which are able to develop further to merozoites and the eryothrozytic stage. Double knocked out Plasmodium organisms reduce said escape or "or break through infection", however, large amounts of sporozoites per immunization are necessary to obtain sufficient immunization. In contrast, in case of the genetically engineered, live attenuated Plasmodium organism according to the present invention wherein the late liver stage specific promotor is present, sporozoites can enter hepatocytes and may propagate therein, however, further development into the merozoites and the eryothrocytic stage is inhibited. Thus, less sporozoites are necessary to allow effective immunization which will also reduce the danger of "break through infection".

The approach of genetic modification of the Plasmodium organisms as described herein may be combined with the known knock out strategy, e.g. as described in WO 2006/062928, thus, providing live attenuated Plasmodium organisms wherein the relevant protein, e.g. the Pb103464 protein has been knocked out, and in addition, the nucleic acid molecules according to the present invention wherein a nucleic acid molecule is under the control of the promotor of the present invention is introduced by genetic engineering.

It is assumed that by using these Plasmodium organisms according to the present invention, in particular, the Plasmodium organisms having additionally a knock out, the risk of entering parasites into the eryothrocytic stage will be inhibited when full attenuation will be achieved.

The following examples illustrate representative embodiments now contemplated for practizising the invention, but should not be constructed to limit the invention.

### Examples

### pL0017-pb103464-promotor plasmid (pPrLSA4) design and transfection

DNA was amplified by polymerase chain reaction (PCR) from *P. berghei* blood stage parasite genomic DNA (gDNA) template using the Phusion Taq DNA polymerase High Fidelity enzyme (Finnzyme). Preparation of gDNA was performed using the Blood DNA Extract Kit (Qiagen). The pbeef1aa promoter of the *P. berghei* transfection plasmid pL0017 was removed by NheI/BamHI digest and replaced by the promotor of *pb103464*. The promotor of *pb103464* was obtained by amplification of *P. berghei* gDNA using the primer set: 599 (5'-CGGCTAGCGTTGCATTATCGTCAAAAGTG-3'), (Seq.ID. No. 3 and 601 (5'-CGGGATCCATGTGTAAAAAAGTAAAATGATTATAATAGAAGTG-3'), Seq.-ID. No.4. The resulting fragment was cloned into the NheI/BamHI cloning sites of pL0017. Schizont-stage *Plasmodium berghei* parasites were transfected with 5µg of purified plasmid DNA (Machery and Nagel Kit PC100), as previously described ( Janse, C.J., Ramesar, J. and Waters, A.P. (2006), Nat Protoc. 1: 346-356. Subsequently, the transfected parasites were injected into the tail vene of a naive mouse. Infected mice were supplied with pyrimethamine in the drinking water, which kills all wildtype parasites and allows only transgenic parasites to survive. Selected parasite strains were investigated for GFP expression in all life cycle stages of the parasite.

Analysis of the transfected cells were done by methods known in the art using standard techniques.

## Claims

1. An isolated liver-stage specific promotor nucleic acid of Seq ID No. 1 or functional fragments or functional derivatives thereof allowing liver-stage specific expression of mRNA in a Plasmodium organism.

2. The isolated nucleic acid according to claim 1 further containing a nucleic acid sequence encoding a heterologous peptide under the control of the liver stage specific promotor.

3. The isolated nucleic acid molecule according to any one of the preceding claims wherein said heterologous peptide is a toxin.

4. The isolated nucleic acid molecule according to claim 3 wherein the toxin is selected from Clostridium perfringens, Listeria monocytogenes, Yersinia pestis, Shigella dysenteriae, Escherichia coli, Plasmodium spec.

5. The isolated nucleic acid molecule according to claim 2 wherein the nucleic acid encoding a heterologous peptide is a Plasmodium-derived nucleic acid encoding a peptide which expression is not restricted to the liver-stage phase.

6. A genetically engineered live Plasmodium organism containing a nucleic acid molecule according to any one claims 1 to 5.

7. The genetically engineered live Plasmodium organism according to claim 6 which is selected from Plasmodium falciparum, Plasmodium vivax, Plasmodium ovale, Plasmodium malariae, Plasmodium berghei, Plasmodium yoelli.

8. Pharmaceutical composition comprising a live Plasmodium organism according to claim 6 or 7, or isolated nucleic acid molecule according to any one of claims 1 to 5, and, optionally, a diluent, carrier or excipient.

9. The pharmaceutical composition of claim 8 which is a vaccine.

10. The pharmaceutical composition of claim 8 or 9 which is a living vaccine suitable for administration via the parenteral route.

11. The pharmaceutical composition of claim 10 suitable for administration via subcutaneous injection of live attenuated Plasmodium organism according to claim 6 or 7.

12. A method of producing genetically engineered live Plasmodium organism suitable for vaccination comprising the step of introducing an isolated nucleic acid molecule of any one of claims 1 to 5 into a live Plasmodium organism and selecting genetically engineered live Plasmodium organism.

13. An isolated nucleic acid molecule of Seq ID No. 2 or functional fragments or derivatives thereof.

14. An isolated polypeptide comprising an amino acid sequence of Seq ID No. 3 and functional, in particular, immunogenic, fragments or derivatives thereof.


SEQUENCE LISTING

<110>  Bernhard-Nocht-Insitut für Tropenmedizin

<120>  Attenuated liver-stage Plasmodium and vaccine containing the same

<130>  4533-001EP-1

<160>  5

<170>  PatentIn version 3.3

<210>  1
<211>  987
<212>  DNA
<213>  Plasmodium berghei

<400>  1
agagttgcat tatcgtcaaa agtgtataaa attataaaat agagcaacat aaaatggata      60

ataatggtga aactaaaaat aataaaatta aagttgggta aataaaaaca caacataaag     120

gatcaaggta ttattttttt aaattttttt tatgaaaaac tattagaaca atttaaaata     180

atgcatatta tgctttattt gtacaatata attaaaataa aacgaataac gatgtgatta     240

ttcaatatgc agattaataa tatatacaca tgtaaatgta tatatacttc ttcattgtca     300

aattccttag gcatatgtag catatatggt ttttgacggt tctttaaaaa tatacaactg     360

taatccctaa taaatgaatg cagttgaaat cgaacaaatc catatgcttc cgtatcgatt     420

taaccattta ggtagtgatc tatgatcgat ctacatgtga agaaaattgg catcttttaa     480

aaactctttc ataatggttt atgagttctt tagatcatat attatttaaa caaaaaataa     540

aataaatcac ctattttttt atattcgata aagcgtgcaa acaaatttgc atgtatgtat     600

tctattatcg ttggtttttt ttacaccacc ttttttttat ttataatgtt tttcaccaca     660

aagcaactat atataaaaaa cacactaaaa atatataatt aaattaaaaa ataataataa     720

agaaaataaa aaataaaaaa taaaaagcat atgtattata catgcatgta tattccctgc     780

atatgtatgt tttcagttca gattaagaac aaaaaaatac agataattaa gcatttattt     840

gttacgttta tacatgtcaa taccaaaaat atatatgtgc ctacacatgt tacatataag     900

tagatacata taaatatatc ggagtaatat tcttcttctt tcatttttca cttctatcat     960

aatcatttta ctttttaaac ataaaaa                                         987


<210>  2
<211>  1167
<212>  DNA
<213>  Plasmodium berghei


<220>
<221>  CDS
<222>  (1)..(1167)

<400>  2
atg aaa gat cat ata aaa aat gta tgc ttt cgg aaa act cta cta att      48
Met Lys Asp His Ile Lys Asn Val Cys Phe Arg Lys Thr Leu Leu Ile
1               5                   10                  15

tca cta ctg ctt att ata tta aaa tac aca aaa tat gat tat tta gaa 96
Ser Leu Leu Leu Ile Ile Leu Lys Tyr Thr Lys Tyr Asp Tyr Leu Glu
            20                  25                  30

aaa gaa aat gat gaa aaa caa aaa tac aac agc aat ata tcg tca cca 144
Lys Glu Asn Asp Glu Lys Gln Lys Tyr Asn Ser Asn Ile Ser Ser Pro
        35                  40                  45

agt tta cta aga aca tat ttt gat gta tat gtc aat agg aaa aat acg 192
Ser Leu Leu Arg Thr Tyr Phe Asp Val Tyr Val Asn Arg Lys Asn Thr
    50                  55                  60

gat gta aag aca ttg aag aaa ttg tta aaa aat gtt acg att tct aat 240
Asp Val Lys Thr Leu Lys Lys Leu Leu Lys Asn Val Thr Ile Ser Asn
65                  70                  75                  80

aat tac ctt tgc atg gtg aaa aag gta aac ata aat gtt tac aat aaa 288
Asn Tyr Leu Cys Met Val Lys Lys Val Asn Ile Asn Val Tyr Asn Lys
                85                  90                  95

ata ttt ggt aat aag atg agt aaa att ata ttg agc aac gaa aga aaa 336
Ile Phe Gly Asn Lys Met Ser Lys Ile Ile Leu Ser Asn Glu Arg Lys
            100                 105                 110

tat ggt aaa aat gat tta aaa aaa aag tgc att act act gaa tct tca 384
Tyr Gly Lys Asn Asp Leu Lys Lys Lys Cys Ile Thr Thr Glu Ser Ser
        115                 120                 125

tcg tgc aca ttt ggt gaa gaa aaa aaa agg aaa aaa tta ttc aca gat 432
Ser Cys Thr Phe Gly Glu Glu Lys Lys Arg Lys Lys Leu Phe Thr Asp
    130                 135                 140

tat gaa aaa ata aat ttg tat aaa aat agt aac ccc ctt gag gat att 480
Tyr Glu Lys Ile Asn Leu Tyr Lys Asn Ser Asn Pro Leu Glu Asp Ile
145                 150                 155                 160

gac gca cat gga aat aca att aaa ggt ttt ctt ttt gaa cta ttt gat 528
Asp Ala His Gly Asn Thr Ile Lys Gly Phe Leu Phe Glu Leu Phe Asp
                165                 170                 175

ata att gta aaa att aag aaa att gta ata gat gca tct aac aaa atg 576
Ile Ile Val Lys Ile Lys Lys Ile Val Ile Asp Ala Ser Asn Lys Met
            180                 185                 190

att tcc gtg tgc cta ttt aat aat gat aat tac gaa gaa aac tta ttc 624
Ile Ser Val Cys Leu Phe Asn Asn Asp Asn Tyr Glu Glu Asn Leu Phe
        195                 200                 205

tac ata aaa aat aat aag gat tat gat gaa ttt cat aat tat aat tct 672
Tyr Ile Lys Asn Asn Lys Asp Tyr Asp Glu Phe His Asn Tyr Asn Ser
    210                 215                 220

tat aaa tat tat gaa aat cca aat ata aat aca tac aac atg aat aaa 720
Tyr Lys Tyr Tyr Glu Asn Pro Asn Ile Asn Thr Tyr Asn Met Asn Lys
225                 230                 235                 240

gaa ttg tat gta tat aat gag gaa tat gct aat aaa tat tta tat tct 768
Glu Leu Tyr Val Tyr Asn Glu Glu Tyr Ala Asn Lys Tyr Leu Tyr Ser
                245                 250                 255

ttt ata gac att tca tct gat aat tta aaa tgt cga ttt tat caa aaa 816
Phe Ile Asp Ile Ser Ser Asp Asn Leu Lys Cys Arg Phe Tyr Gln Lys
            260                 265                 270

tct gtt caa gga gaa gat aga aat ata gtt cct gat att ttt aag aat 864
Ser Val Gln Gly Glu Asp Arg Asn Ile Val Pro Asp Ile Phe Lys Asn
        275                 280                 285

aaa aat gcg tat aat tta tct aag gat aaa aag tat gat aac cat agt 912
Lys Asn Ala Tyr Asn Leu Ser Lys Asp Lys Lys Tyr Asp Asn His Ser
    290                 295                 300

aag atg aat gat gga aat aaa tcg aat tgt ggg gtt tca agt gac aaa 960
Lys Met Asn Asp Gly Asn Lys Ser Asn Cys Gly Val Ser Ser Asp Lys
305                 310                 315                 320

acc gaa aaa tat ata ttc gac act gaa aca aat aga aac ata aaa ctt 1008
Thr Glu Lys Tyr Ile Phe Asp Thr Glu Thr Asn Arg Asn Ile Lys Leu
                325                 330                 335

aaa aag gaa caa ttt ttg gaa aca aat gca aaa tgg aat gaa agc aaa 1056
Lys Lys Glu Gln Phe Leu Glu Thr Asn Ala Lys Trp Asn Glu Ser Lys
            340                 345                 350

ata aac caa tca aac aaa ctg cac aaa aaa aat gac gaa gca aaa tat 1104
Ile Asn Gln Ser Asn Lys Leu His Lys Lys Asn Asp Glu Ala Lys Tyr
        355                 360                 365

agg tcg ata gga aga att tta gca gaa act caa cat gaa aat tta gtt 1152
Arg Ser Ile Gly Arg Ile Leu Ala Glu Thr Gln His Glu Asn Leu Val
    370                 375                 380

tta agt aca gat taa 1167
Leu Ser Thr Asp
385

<210> 3
<211> 388
<212> PRT
<213> Plasmodium berghei

<400> 3

Met Lys Asp His Ile Lys Asn Val Cys Phe Arg Lys Thr Leu Leu Ile
1               5                   10                  15

Ser Leu Leu Leu Ile Ile Leu Lys Tyr Thr Lys Tyr Asp Tyr Leu Glu
            20                  25                  30

Lys Glu Asn Asp Glu Lys Gln Lys Tyr Asn Ser Asn Ile Ser Ser Pro
        35                  40                  45

Ser Leu Leu Arg Thr Tyr Phe Asp Val Tyr Val Asn Arg Lys Asn Thr
    50                  55                  60

Asp Val Lys Thr Leu Lys Lys Leu Leu Lys Asn Val Thr Ile Ser Asn
65                  70                  75                  80

Asn Tyr Leu Cys Met Val Lys Lys Val Asn Ile Asn Val Tyr Asn Lys
                85                  90                  95

Ile Phe Gly Asn Lys Met Ser Lys Ile Ile Leu Ser Asn Glu Arg Lys
            100                 105                 110

Tyr Gly Lys Asn Asp Leu Lys Lys Lys Cys Ile Thr Thr Glu Ser Ser
        115                 120                 125

Ser Cys Thr Phe Gly Glu Glu Lys Lys Arg Lys Lys Leu Phe Thr Asp
    130                 135                 140

Tyr Glu Lys Ile Asn Leu Tyr Lys Asn Ser Asn Pro Leu Glu Asp Ile
145                 150                 155                 160

Asp Ala His Gly Asn Thr Ile Lys Gly Phe Leu Phe Glu Leu Phe Asp
                165                 170                 175

Ile Ile Val Lys Ile Lys Lys Ile Val Ile Asp Ala Ser Asn Lys Met
            180                 185                 190

Ile Ser Val Cys Leu Phe Asn Asn Asp Asn Tyr Glu Glu Asn Leu Phe
        195                 200                 205

Tyr Ile Lys Asn Asn Lys Asp Tyr Asp Glu Phe His Asn Tyr Asn Ser
    210                 215                 220

Tyr Lys Tyr Tyr Glu Asn Pro Asn Ile Asn Thr Tyr Asn Met Asn Lys
225                 230                 235                 240

Glu Leu Tyr Val Tyr Asn Glu Glu Tyr Ala Asn Lys Tyr Leu Tyr Ser
                245                 250                 255

Phe Ile Asp Ile Ser Ser Asp Asn Leu Lys Cys Arg Phe Tyr Gln Lys
            260                 265                 270

Ser Val Gln Gly Glu Asp Arg Asn Ile Val Pro Asp Ile Phe Lys Asn
        275                 280                 285

Lys Asn Ala Tyr Asn Leu Ser Lys Asp Lys Lys Tyr Asp Asn His Ser
    290                 295                 300

Lys Met Asn Asp Gly Asn Lys Ser Asn Cys Gly Val Ser Ser Asp Lys
305                 310                 315                 320

Thr Glu Lys Tyr Ile Phe Asp Thr Glu Thr Asn Arg Asn Ile Lys Leu
                325                 330                 335

Lys Lys Glu Gln Phe Leu Glu Thr Asn Ala Lys Trp Asn Glu Ser Lys
            340                 345                 350

Ile Asn Gln Ser Asn Lys Leu His Lys Lys Asn Asp Glu Ala Lys Tyr
        355                 360                 365

Arg Ser Ile Gly Arg Ile Leu Ala Glu Thr Gln His Glu Asn Leu Val
    370                 375                 380

Leu Ser Thr Asp
385

<210>  4
<211>  29
<212>  DNA
<213>  artificial

<220>
<223>  primer sense

<400>  4
cggctagcgt tgcattatcg tcaaaagtg                                          29


<210>  5
<211>  43
<212>  DNA
<213>  artificial

<220>
<223>  primer antisense

<400>  5
cgggatccat gtgtaaaaaa gtaaaatgat tataatagaa gtg                          43